(19) Europäisches Patentamt
European Patent Office
Office européen des brevets

(11) EP 0 910 369 B1

(12) **EUROPEAN PATENT SPECIFICATION**

(45) Date of publication and mention of the grant of the patent:
**21.04.2010 Bulletin 2010/16**

(21) Application number: **97915139.6**

(22) Date of filing: **20.03.1997**

(51) Int Cl.:
***A61K 31/38*** *(2006.01)* ***C07D 333/56*** *(2006.01)*

(86) International application number:
**PCT/US1997/004259**

(87) International publication number:
**WO 1997/035571 (02.10.1997 Gazette 1997/42)**

(54) **BENZOTHIOPHENES, FORMULATIONS CONTAINING SAME, AND METHODS**

BENZOTHIOPHENE UND DENEN ENTHALTENDE ZUSAMMENSETZUNGEN

BENZOTHIOFENES, FORMULATIONS DANS LESQUELLES ILS ENTRENT ET TECHNIQUES

(84) Designated Contracting States:
**AT BE CH DE DK ES FI FR GB GR IE IT LI LU NL PT SE**
Designated Extension States:
**AL LT LV RO SI**

(30) Priority: **26.03.1996 US 14167 P**
**04.04.1996 GB 9607110**

(43) Date of publication of application:
**28.04.1999 Bulletin 1999/17**

(60) Divisional application:
**09165873.2**

(73) Proprietor: **ELI LILLY AND COMPANY**
**Indianapolis, IN 46285 (US)**

(72) Inventors:
- **ARBUTHNOT, Gordon, N.**
**Indianapolis, IN 46256 (US)**
- **DALDER, Brian, W.**
**West Lafayette, IN 47906 (US)**
- **HARTAUER, Kerry, J.**
**Carmel, IN 46032 (US)**
- **LUKE, Wayne, D.**
**West Lafayette, IN 47906 (US)**
- **STRATFORD, Robert, E., Jr.**
**Carmel, IN 46032 (US)**

(74) Representative: **Vaughan, Jennifer Ann et al**
**Eli Lilly and Company Limited**
**Lilly Research Centre**
**Erl Wood Manor**
**Windlesham, Surrey GU20 6PH (GB)**

(56) References cited:
**US-A- 5 494 920** **US-A- 5 494 929**
**US-A- 5 532 254**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 0 910 369 B1

Printed by Jouve, 75001 PARIS (FR)

## Description

This invention relates to the fields of pharmaceutical and organic chemistry and provides a benzothiophene compound, in particulate form, which is useful for the treatment of various medical indications, including osteoporosis and lipid lowering. More particularly, the benzothiophene is of a particle size range which allows enhanced bioavailability and control during the manufacturing process.

Osteoporosis describes a group of diseases which arise from diverse etiologies, but which are characterized by the net loss of bone mass per unit volume. The consequence of this loss of bone mass and resulting bone fracture is the failure of the skeleton to provide adequate structural support for the body. One of the most common types of osteoporosis is that associated with menopause. Most women lose from about 20% to about 60% of the bone mass in the trabecular compartment of the bone within 3 to 6 years after the cessation of menses. This rapid loss is generally associated with an increase of bone resorption and formation. However, the resorptive cycle is more dominant and the result is a net loss of bone mass. Osteoporosis is a common and serious disease among postmenopausal women.

Raloxifene is now in Phase III clinical trials for osteoporosis. Indications thus far from these trials and other data, point to raloxifene's potential not only as an osteoporosis therapy, but also of potential use in lowering LDL (serum lipid) levels, inhibiting endometriosis and uterine fibrosis, and preventing breast cancer. The advancement of raloxifene has been somewhat hampered by its physical characteristics, both as to bioavailability and in manufacturing. For example, it is generally insoluble, and this can adversely affect the bioavailability. Clearly, any improvement in the physical characteristics of raloxifene, would potentially offer a more beneficial therapy and enhanced manufacturing capability. US 5,494,920 is directed to 2-phenyl-3-aroylbenzothiophenes useful for inhibiting viral replication. US 5,494,929 is directed to 2-phenyl-3-aroylbenzothiophenes useful for inhibiting growth hormone effects. US 5,532,254 is directed to 2-aryl-3-aroyl benzo[b]thiophenes effective in modulating calcium channels.

This invention provides a compound of formula I

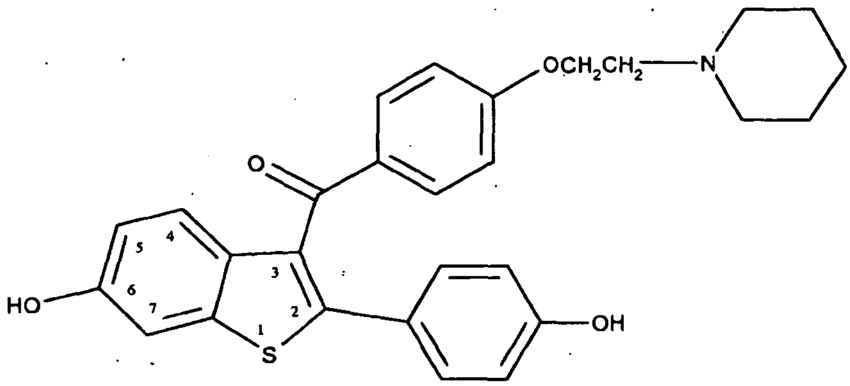

(I)

and pharmaceutically acceptable salts and solvates thereof, **characterized in that** the compound is in particulate form, said particles having a mean particle size of less than about 25 microns, and preferably between about 5 and about 20 microns, at least about 90% of said particles having a size of less than about 50 microns.

Further, the present invention encompasses compounds of formula I wherein at least 90% of the particles have a particle size of less than about 35 microns.

The present invention further relates to pharmaceutical compositions containing one or more compounds of formula I, optionally containing estrogen or progestin, and the use of such compounds, alone, or in combination with estrogen or progestin, for alleviating the symptoms of osteoporosis lowering lipid levels, and inhibiting endometriosis, uterine fibrosis, and breast cancer.

It has now been found that by processing compounds of formula I, to bring their particle size within a specified narrow range, pharmaceutical compositions may be prepared which exhibit for their active ingredient both a consistent *in vitro* dissolution profile and *in vivo* bioavailability. In addition to ringing about these desired dissolution/bioavailability characteristics, the control of particle size to a narrow range has also resulted in significant improvements in manufacturing capabilities.

The mean particle size of the compounds of formula I, as set out by the invention, is less than about 25 microns, preferably between about 5 and about 20 microns, with at least 90% of the particles having a particle size of less than about 50 microns, preferably less than about 35 microns. More preferably, the mean particle size range is between about

5 and about 20 microns, with at least 90% of the particles having a size of less than about 35 microns.

It will of course be understood by those familiar with comminution process techniques that the limit set on the size of 90% or more of the particles is a limitation to further distinguish the particulate compounds of the invention from those exhibiting a broader size distribution, because of the wide variation in size encountered in all matter reduced in size by a process of comminution or particle size reduction, for example, by milling utilizing a variety of kinds of milling equipment now available, for example, hammer, pin or fluid energy mills.

The invention also provides pharmaceutical compositions comprising the said particulate compound of the invention and one or more pharmaceutically-acceptable excipients or carriers.

The term "solvate" represents an aggregate that comprises one or more molecules of the solute, such as a formula I compound, with a molecule of solvent. Representative solvates are formed with methylene chloride, 1,2-dichloroethane, chloroform, and 1,2,3-trichloropropane.

Raloxifene's chemical name is 6-hydroxy-2-(4-hydroxyphenyl)-3-[4-(2-piperidinoethoxy)benzoyl]benzo[b]-thiophene. "Raloxifene" also encompasses the salts and solvates thereof, with the hydrochloride salt being preferred.

The compounds of the current invention can be made according to established procedures, such as those detailed in U.S. Patent Nos. 4,133,814, 4,418,068, and 4,380,635

A preferred form of raloxifene hydrochloride for use in the invention is the non-solvated, crystalline form described in UK Patent Application No. 2293382, or German Patent Specification No. 19534744, having the x-ray diffraction characteristics specified therein.

Often, compounds which have poor solubility profiles can have their bioavailability enhanced by increasing the surface area of the formulated particles. The surface area generally increases per unit volume as the particle size decreases. Various techniques for grinding or milling a drug substance are well known in the art and each of these techniques are commonly used to decrease particle size and increase the surface area of the particle. It would seem reasonable that the best way to deal with any slightly soluble compound would be to mill it to the smallest size possible; however, this is not always practical or desirable. The milling process has an economic cost not only it the direct cost of the process, itself, but also with other associated factors. For example, very finely divided material presents difficulties and cost in capsule filling or tablet preparation, because the material will not flow, but becomes caked in finishing machinery. Such finishing difficulties generate non-homogeneity in the final product, which is not acceptable for a drug substance. Additionally, the milling process, physically generates heat and pressure on the material, such conditions lead to chemical degradation of the compound, thus such milling techniques are usually kept to a minimum.

Therefore, there is always dynamic between the properties which yield the maximum bioavailability (particle surface area) and the practical limits of manufacture. The point of compromise which marks this "best solution" is unique to each situation and unique as to its determination.

Methods for determining the size of particles are known in the art. The following is a description of one method, but is not intended to be limiting. For example, the general method of U.S. Patent No. 4,605,517 could be employed.

In preparing the particulate compound of the invention a compound of formula I, in its raw state, is first characterized for size using an instrument adapted to measure equivalent spherical volume diameter, that is to say a Horiba LA900 Laser Scattering Particle Size Distribution Analyzer or equivalent instrument. Typically a representative sample of a compound of formula I would be expected to comprise in its raw state particles having a mean equivalent spherical volume diameter of about 110-200 microns and with a broad size distribution.

After being characterized for size in its raw state, the raw compound is then milled, preferably using a pin mill under suitable conditions of mill rotation rate and feed rate, to bring the particle size value within the above mentioned limits according to the invention. The efficiency of the milling is checked by sampling using a Horiba LA900 Laser Scattering Particle Size Distribution Analyzer and the final particle size is checked in a similar manner. If the first pass through the mill does not produce the required size distribution, then one or more further passes are effected.

The compound of formula I in its particulate form within the above mentioned limits according to the invention may then be mixed with an excipient or carrier as necessary and, for example, compressed into tablets. Thus, for example, the particulate compound may be mixed with anhydrous lactose, lactose monohydrate, cross povidone and granulated in an aqueous dispersion of povidone and polysorbate 80. After drying and milling into granules the material can be terminally blended with magnesium stearate and compressed into tablets.

Because the particles in the raw state as well as after milling or other particle size reduction techniques are irregular in shape, it is necessary to characterize them not by measurement of an actual size such as thickness or length, but by measurement of a property of the particles which is related to the sample property possessed by a theoretical spherical particle. The particles are thus allocated an "equivalent spherical diameter".

The values found from characterizing a large number of "unknown" particles can be plotted frequency vs. diameter or in other methods weight vs. diameter, usually adopting percentage undersize values for frequency or weight. This gives a characteristic curve representing size distribution of the sample, i.e., cumulative percentage undersize distribution curve. Values from this can be read off directly or plotted on log-probability paper to give an appropriate straight line. The mean equivalent spherical volume diameter is the 50% undersize value.

The mean equivalent spherical volume diameter found is thus a statistical representation of a theoretical particle having the same property as the "unknown" particle.

As indicated above the mean equivalent sphere volume diameter of the particles of the milled compound of formula I may be evaluated using a Horiba LA900 Laser Scattering Particle Size Distribution Analyzer. Using such an instrument values for a suspension of the particle of unknown size may be obtained and the instrument may be monitored using a control sample having particles within the size range expected based on statistical analysis of the sample. Multiple runs of the control sample established the standard deviation in measurement of the mean to be 1.3 microns.

Following is a description by way of example of the preparation of compositions in accordance with the invention. In all of the Examples the compound was prepared from raw form using a pin mill and consisted of particles having a mean equivalent spherical volume diameter of between about 5 and 20 microns, at least 90% of the particles having a particle size of less than about 35 microns.

The particle size of the reduced raloxifene HCl was measured as follows. The laser scattering particle size distribution analysis was effected on a small sample of the reduced material which is suspended in approximately 180 ml of dispersant solution. Sample is added to the dispersant until an acceptable level of laser light obscuration achieved at which point the particle size distribution is measured. Prior to the sample suspension the dispersant solution was prepared by adding 20 drops of Coulter 1A dispersant to a saturated aqueous solution of raloxifene HCl. The dispersant solution was filtered through a 0.2 micron microporous membrane filter to provide the necessary particle-free suspending dispersant.

Within five minutes of the preparation of the dispersion, triplicate particle size measurements were performed. Triplicate measurements are effected as a minimum check a) to produce more reliable measurements and b) to check the equivalent sampling of the suspended material has been reproducible i.e., the suspension has not settled.

The results were automatically recorded and displayed graphically to give a frequency percentage vs. undersize and a cumulative percentage vs. undersize characteristic curves for the sample. From this, the mean equivalent spherical volume diameter value was derived (50% undersize value) together with the standard deviation of the distribution calculated as above.

Several physical properties of raloxifene hydrochloride have been investigated during the progression of the compound through development. These include particle size, surface area, and powder bulk density.

A primary determinant in the potential influence of such properties on drug product performance is the aqueous solubility of the drug substance. Raloxifene hydrochloride has a water solubility of approximately 0.3 mg/mL at 25°C and significantly lower values in Simulated Gastric Fluid, USP (0.003 mg/mL) and Simulated Intestinal Fluid, USP (0.002 mg/mL) at 37°C. The aqueous value falls into the USP classification of "very slightly soluble", while according to the recent SUPAC guidance ("Industry Guidance Immediate Release Solid Oral Dosage Forms Pre- and Post-Approval Changes: Chemistry, Manufacturing and Controls, In Vitro Dissolution Testing, and In Vivo Bioequivalence Documentation", Prepared by the Immediate Release Scale-Up and Post Approval Change (SUPAC), Expert Working Group of the Chemistry Manufacturing Controls Coordinating Committee (CMC CC) of the Center for Drug Evaluation and Research at the FDA) on immediate release solid oral dosage forms, the compound has low solubility with a dose solubility volume of greater than 250 mL. Given the low solubility, the rate at which the dosage form dissolves in the gastrointestinal tract can potentially impact the rate and extent of absorption of the active compound. Two related physical properties of the bulk drug which can alter the dissolution rate of the dosage form are particle size and surface area. The impact of surface area which is a function of particle size is illustrated in the Noyes-Whitney equation given below.

$$dC/dt = (D/h) * (S) * (Cs-C)$$

Here, *C* is the concentration of drug at time *t*, *D* is the diffusion coefficient of drug in the medium, *h* is the thickness of diffusion layer, *Cs* is the saturation solubility of drug in the diffusion layer and S is the effective surface area of the drug particles. To ascertain the effect of particle size/surface area of raloxifene HCl on *in vitro* dissolution, lots with varying particle size distributions were obtained via recrystallization and further modified through various milling technologies. The following table contains pertinent data on four bulk lots produced in this effort, which includes particle size data generated utilizing laser light diffraction, and surface area data collected by nirogen adsorption, and analyzed through the BET (Brunauer, Emmett, Teller) equation.

**Table 6**

| Bulk Lot # | Milling Technology | Surface Area $m^2$/gm | Mean Particle Size (μm) | 90% less than (μm) |
|---|---|---|---|---|
| #1 | Micronized | 6.09 | 3.9 | 6.8 |
| #2 | Recrystallized | 2.28 | 8.4 | 13.9 |
| #3 | Ball Milled | 2.10 | 23.3 | 55.3 |

(continued)

| Bulk Lot # | Milling Technology | Surface Area $m^2$/gm | Mean Particle Size (μm) | 90% less than (μm) |
|---|---|---|---|---|
| #4 | Slurry Milled | 0.45 | 48:1 | 89 |

These four bulk lots were handfilled into capsules to provide 60.0 mg of raloxifene hydrochloride and submitted for dissolution testing in a 0.1% aqueous polysorbate 80 medium utilizing USP Apparatus II, with a paddle speed of 50 rpm. Data was collected at 10, 20, 30 and 45 minutes to produce a dissolution profile.

**Table 7**

| Lot # 1 (micronized) | | Lot #2 (Control) | |
|---|---|---|---|
| Time (min.) Dissolved | % Dissolved | Time (min.) | % |
| 10 | 51 | 10 | 41 |
| 20 | 68 | 20 | 60 |
| 30 | 78 | 30 | 68 |
| 45 | 88 | 45 | 74 |
| Lot #3 (Ball-milled) | | Lot #4 (Slurry Milled) | |
| Time (min.) Dissolved | % Dissolved | Time (min.) | % |
| 10 | 31 | 10 | 15 |
| 20 | 45 | 20 | 27 |
| 30 | 54 | 30 | 35 |
| 45 | 64 | 45 | 49 |

It was observed that a range of dissolution profiles resulted from the various particle size distributions of the bulk drug substance, with values ranging from 25% to approximately 80% dissolved at 30 minutes. In an attempt to evaluate these differences upon *in vivo* absorption, a study was conducted in Fischer 344 rats. In the study, rats were dosed with the same four bulk raloxifene lots in their diet (0.4% w/w) for seven days. Plasma concentrations of unconjugated raloxifene were quantitated by HPLC for the four bulk lots. The following table shows the excellent linear correlations obtained between the percent raloxifene hydrochloride dissolved at 10 minutes or 30 minutes in the *in vitro* dissolution test to the average area under the curve (AUC, ng-h/mL) values obtained in rats for each of the bulk drug lots.

**Table 8**

| Lot | % Dissolved at 10 Minutes | % Dissolved at 30 Minutes | AUC (ng-h/mL) |
|---|---|---|---|
| #1 - Micronized | 50 | 78 | 10056 |
| #2 - As Recrystallized | 41 | 68 | 8037 |
| #3 - Ball Milled | 31 | 55 | 5743 |
| #4 - Slurry Milled | 15 | 35 | 3329 |

This *in vitro* to *in-vivo* correlation supports the discriminating ability of the dissolution method, as well as emphasizing the need for a control strategy for either the particle size distribution or surface area of the bulk drug substance. Further evaluation of this data indicated that the particle size data correlates better to the differences noted in the dissolution data and *in vivo* absorption. This can be explained based upon the Noyes-Whitney equation, which relates dissolution to the effective surface area. It is postulated that the surface area as measured by nitrogen adsorption for the various types of milled raloxifene does not predict the effective surface area accessible to the dissolution medium. This is demonstrated when comparing the recrystallized (control) lot (lot #2) and ball milled lot (lot #3). While they have very similar surface area values, 2.28 and 2.10 $m^2$/gm respectively, the recrystallized lot has a finer mean particle size, 8.4 microns compared to 23.3 microns for the ball milled lot. SEM photomicrographs of the ball milled particles show very irregular surfaces with numerous cracks and fissures which would result in increased surface area as measured by nitrogen adsorption, but may not provide surface area accessible to the dissolution medium, resulting in a lower effective surface area. This reasoning can explain the better correlation of the differences in particle size to the differences in *in vitro* dissolution and *in vivo* absorption, compared to the similarity of the surface areas for the two lots. Based upon these findings, the decision was made to pursue particle size distribution as a control parameter to ensure consistent performance of the drug product with regards to release of the drug component.

To further investigate the impact of particle size of raloxifene HCl on drug product performance as measured by *in vitro* dissolution and *in vivo* absorption, a single dose, plasma concentration versus time study was designed in cynomolgus monkeys. The study compared absorption from two bulk lots of raloxifene which possessed mean particle sizes of 48.1 and 9.0 microns. The lots were formulated into granulation matrices representative of granulations being compacted into tablets for human use. In addition, the bulk lot with the 9.0 mean particle size was generated through pin milling technology which represents the desired commercial milling route. The table below summarizes the particle size data of the two bulk lots.

**Table 9**

| Bulk Lot/ Granulation Lot # | Milling Technology | 10% Less Than µm | 50% Less Than µm | 90% Less Than µm | Mean Particle Size |
|---|---|---|---|---|---|
| Lot 5A | Slurry | 11.4 | 44.1 | 90 | 48.1 |
| Lot 5B | Pin | 3.2 | 8.6 | 15.1 | 9.0 |

For the purposes of producing a dissolution profile, the granulations were handfilled into capsules to provide the equivalent of 60 mg of raloxifene hydrochloride. The dissolution data produced in 0.1% aqueous Tween medium, utilizing the paddle method at 50 rpm, are shown below.

**Table 10**

Lot 5A (slurry milled)

| Time (min.) | % Dissolved |
|---|---|
| 10 | 33 |
| 20 | 55 |
| 30 | 65 |
| 45 | 74 |

Lot 5B (pin milled)

| Time (min.) | % Dissolved |
|---|---|
| 10 | 63 |
| 20 | 91 |
| 30 | 95 |
| 45 | 97 |

These differences in particle size distribution again produced significant differences in the dissolution profile in the aqueous 0.1% polysorbate 80 dissolution medium. In the study monkeys received each formulation according to a crossover study design and incorporating a replicate period to allow for intrasubject variability. The following Table 11 shows the mean average plasma concentrations of total raloxifene after the administration of a 25 mg/kg oral dose to the monkeys.

**Table 11**

Lot 5A (slurry milled)

| Time (hrs.) | ng Total Raloxifene/ml plasma |
|---|---|
| 1.4 | 78 |
| 3.6 | 67 |
| 8.2 | 81 |
| 12.1 | 60 |
| 24.3 | 45 |
| 30 | 32 |
| 36.4 | 22 |
| 48.6 | 14 |

(continued)

Lot 5B (pin milled)

| Time (hrs.) | ng Raloxifene/ml plasma |
|---|---|
| 1.4 | 108 |
| 3.6 | 84 |
| 8.2 | 121 |
| 12.1 | 95 |
| 24.3 | 70 |
| 30 | 51 |
| 36.4 | 34 |
| 48.6 | 23 |

As seen from the plasma concentration versus time profiles given in Table 11, the formulation with the finer particle size bulk drug substance provided higher plasma concentrations of total raloxifene at all of the timepoints sampled. The superior absorption from the formulation with the finer particle size is reflected in both the rate and extent of absorption as illustrated in the following summary of pharmacokinetic parameters from the study.

| Lot Number | Cmax (ng/mL) | AUC (ng-h/mL) |
|---|---|---|
| 5B (9.0 microns) | 131 | 3608 |
| 5A (48.1 microns) | 96 | 2357 |

The differences shown were found to be significant upon statistical analysis (AUC, $p<0.005$ and Cmax, $p<0.02$). This data is further evidence of the critical nature of the particle size distribution on its impact on bioavailability. The study also confirms the discriminating ability of the *in vitro* dissolution method and its relationship to *in vivo* absorption. Once again, the differences observed in the *in vitro* dissolution profiles translated into *in vivo* absorption differences.

Based upon the above work and physical property data generated, a particle size specification was established. The invention provides that the mean particle size, as determined by laser light diffraction, should be less than about 25 microns. In addition, 90% of the particles by volume should be under 50 microns, which allows for characterization of the distribution. Preferably, the size is between about 5 and about 20 microns, and 90% of the particles have a size of less than about 35 microns. To justify this range, bulk lots were produced by pin milling and samples of the available extremes were manufactured into formulated tablets and *in vitro* dissolution testing. In one study, six bulk lots of raloxifene hydrochloride (ca. 1 kg) were received and manufactured into formulated 60 mg raloxifene HCl tablets representative of the tablets being utilized in Phase III clinical testing. The particle size data for the lots utilized is summarized in the following Table 12.

**Table 12**

(all particle size in microns)

| Lot # | 10% Less Than | 50% Less Than | 90% Less Than | Mean |
|---|---|---|---|---|
| 6 | 2 | 6 | 12 | 6 |
| 7 | 3 | 8 | 21 | 10 |
| 8 | 3 | 11 | 31 | 15 |
| 9 | 3 | 12 | 30 | 14 |
| 10 | 2 | 5 | 10 | 6 |
| 11 | 3 | 9 | 23 | 11 |

The dissolution profile in 0.1% aqueous polysorbate 80 for all of these 6 bulk lots formulated into tablets are comparable in all cases. In addition, all lots displayed a relatively fast dissolution profile, with values greater than 90% dissolved at 20 minutes. Table 13 sets out the data.

**Table 13**

% Dissolved - Raloxifene Hydrochloride from Core Tablets

| | Lot Number | | | | | |
|---|---|---|---|---|---|---|
| | 6 | 7 | 8 | 9 | 10 | 11 |
| Time (Min) | | | | | | |
| 10 | 89.2 | 88.1 | 81.1 | 74.5 | 84.1 | 80.5 |
| 20 | 92.3 | 92.6 | 95.4 | 91.3 | 96.0 | 93.7 |
| 30 | 93.2 | 93.9 | 97.0 | 93.3 | 96.3 | 94.0 |
| 45 | 93.0 | 94.1 | 98.4 | 93.9 | 96.1 | 94.6 |

To statistically assess the dissolution as a function of particle size, JMP Statistical and Graphics Guide Software (SAS Institute, Inc., Cary, North Carolina) was utilized and a plot was generated where the percent dissolved at 20 minutes was plotted as a function of the average particle size of each lot.

The scatter observed in the plot, along with the high p-value (0.81) support the conclusion of a non-significant effect of particle size on dissolution over this range of particle sizes. Similar analyses were performed at the other timepoint, 10, 30 and 45 minutes, with calculated p-values of 0.11, 0.76, and 0.40 respectively. These high p-values along with the observation of both negative and positive slopes at the various timepoints again support the appropriateness of the range for the particle size.

Another similar study was performed with 7 different particle size distributions of bulk drug, with each again being formulated into 60 mg tablets. The particle size data for these lots is summarized in Table 14.

**Table 14**

(all particle size in microns)

| Lot | 10% Less Than | 50% Less Than | 90% Less Than | Mean |
|---|---|---|---|---|
| 70B | 3.3 | 14.5 | 39.3 | 18.8 |
| 70E | 2.8 | 10.5 | 26.3 | 13.0 |
| 70F | 3.4 | 16.0 | 50.2 | 22.9 |
| 71B | 3.1 | 12.9 | 38.9 | 17.8 |
| 71D | 2.8 | 10.1 | 25.6 | 12.6 |
| 71G | 3.3 | 14.6 | 42.1 | 19.6 |
| 71H | 2.9 | 11.1 | 28.2 | 13.7 |

The dissolution data collected in 0.1% aqueous polysorbate 80 for these seven bulk lots formulated into tablets is given in the following table.

**Table 15**

% Dissolved - Raloxifene Hydrochloride

| | Lot Number | | | | | | |
|---|---|---|---|---|---|---|---|
| | **70B** | **70E** | **70F** | **71B** | **71D** | **71G** | **71H** |
| **Time (Min)** | | | | | | | |
| 10 | 76 | 81 | 73 | 76 | 75 | 61 | 68 |
| 20 | 94 | 96 | 91 | 93 | 88 | 85 | 91 |
| 30 | 98 | 99 | 95 | 98 | 91 | 88 | 95 |
| 45 | 99 | 99 | 97 | 99 | 97 | 97 | 98 |

As with the previous set of particle size distributions, the comparable dissolution profiles obtained with these particle size distributions support the ranges for particle size given in this invention. Given the relationship shown between *in vitro* dissolution and *in vivo* absorption, it follows that the particle size distribution range claimed in this patent will provide surprisingly consistent *in vivo* absorption/bioavailability characteristics.

In addition to the role of particle size *in vitro* dissolution and *in vivo* absorption, another important aspect is its role on the various unit operations of the drug product manufacturing process. While the particle size specification ensures consistent delivery of the drug molecule to the sites of absorption in the gastrointestinal tract, it also allows for better control during the wet granulation step of the tablet manufacturing process. By controlling the particle size, the

variations in quantity of water needed to elicit the appropriate progression of the granulation power consumption curve is reduced. By maintaining the particle size within the previous mentioned constraints, established quantities of water can be dictated in the manufacturing ticket for routine lot manufacture. The granulation step is common to many tablet and capsule manufacturing operations and is typically driven by the addition of water to bring about the desired endpoint of the granulation. A downstream unit operation dependent upon the granulation endpoint is the milling of the dried granulation and the resulting particle size distribution obtained on the granulation. It has been discovered that the incoming particle size of the active ingredient also effects the ultimate particle size distribution of the dry milled agglomerates formed during granulations. For a fixed water quantity, a coarser distribution will result in a finer size distribution of the dry milled agglomerates. Too fine a granulation distribution can lead to poor granulation flow and poor control of individual tablet weight during the compression step. Thus the narrow particle size constraints previously mentioned have also resulted in making the process more amenable to automation by reducing the variations in water required during the granulation step and producing dry milled granules of the appropriate distribution to prevent the rejection of tablets during compression due to unacceptable tablet weight.

The present invention also provides compounds of Formula I for use in inhibiting osteoporosis, treating or prevent breast cancer, inhibiting uterine fibrosis, inhibiting endometriosis, and lowering serum cholesterol.

As used herein, the term "effective amount" means an amount of compound of formula I which is capable of alleviating the symptoms of the various pathological conditions herein described. The specific dose of a compound administered according to this invention will, of course, be determined by the particular circumstances surrounding the case including, for example, the compound administered, the route of administration, the state of being of the patient, and the pathological condition being treated. A typical daily dose will contain a nontoxic dosage level of from about 10.0 mg to about 1000 mg/day of a compound of the present invention. Preferred daily doses generally will be from about 50 mg to about 150 mg/day.

Besides the hydrochloride salt, the compounds of this invention form pharmaceutically acceptable acid and base addition salts with a wide variety of organic and inorganic acids and bases and include the physiologically acceptable salts which are often used in pharmaceutical chemistry. Such salts are also part of this invention. Typical inorganic acids used to form such salts include hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzene-sulfonate, p-bromophenylsulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like. Of course, the preferred salt is the hydrochloride salt.

The pharmaceutically acceptable acid addition salts are typically formed by reacting a compound of formula I with an equimolar or excess amount of acid.

The compounds of this invention can be administered by a variety of routes including oral, rectal, transdermal, subucutaneus, intravenous, intramuscular, and intranasal. These compounds preferably are formulated prior to administration, the selection of which will be decided by the attending physician. Thus, another aspect of the present invention is a pharmaceutical composition comprising an effective amount of a compound of Formula I, or a pharmaceutically acceptable salt thereof, optionally containing an effective amount of estrogen or progestin, and a pharmaceutically acceptable carrier, diluent, or excipient.

The total active ingredients in such formulations comprises from 0.1% to 99.9% by weight of the formulation. By "pharmaceutically acceptable" it is meant the carrier, diluent, excipients and salt must be compatible with the other ingredients of the formulation, and not deleterious to the recipient thereof.

Pharmaceutical formulations of the present invention can be prepared by procedures known in the art using well known and readily available ingredients. For example, the compounds of formula I, with or without an estrogen or progestin compound, can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl-pyrrolidone; moisturizing agents such as glycerol; disintegrating agents such as calcium carbonate, sodium bicarbonate and cross-linked povidone (cross povidone); agents for retarding dissolution such as paraffin; resorption accelerators such as quaternary ammonium compounds; surface active agents such as cetyl alcohol, polysorbate 80, glycerol monostearate; adsorptive carriers

such as kaolin and bentonite; and lubricants such as talc, calcium and magnesium stearate, and solid polyethyl glycols.

The compounds also can be formulated as elixirs or solutions for convenient oral administration or as solutions appropriate for parenteral administration, for example, by intramuscular, subcutaneous or intravenous routes. Additionally, the compounds are well suited to formulation as sustained release dosage forms and the like. The formulations can be so constituted that they release the active ingredient only or preferably in a particular physiological location, possibly over a period of time. The coatings, envelopes, and protective matrices may be made, for example, from polymeric substances or waxes.

Compounds of formula I, alone or in combination with another pharmaceutical agent, generally will be administered in a convenient formulation. The following formulation examples only are illustrative and are not intended to limit the scope of the present invention.

Formulations

In the formulations which follow, raloxifene HCl has a particulate size as set out by the invention.

Formulation 1: Gelatin Capsules

Hard gelatin capsules are prepared using the following:

| Ingredient | Quantity (mg/capsule) |
|---|---|
| Raloxifene HCl | 10.0 - 1000 |
| Starch, NF | 0 - 650 |
| Starch flowable powder | 0 - 650 |
| Silicone fluid 350 centistokes | 0 - 15 |

The formulation above may be changed in compliance with the reasonable variations provided.

A tablet formulation is prepared using the ingredients below:

Formulation 2: Tablets

| Ingredient | Quantity (mg/tablet) |
|---|---|
| Raloxifene HCl | 2.5 - 1000 |
| Cellulose, microcrystalline | 200 - 650 |
| Silicon dioxide, fumed | 10 - 650 |
| Stearic acid | 5 - 15 |

The components are blended and compressed to form tablets.

Alternatively, tablets each containing 2.5 - 1000 mg of Raloxifene are made up as follows:

Formulation 3: Tablets

| Ingredient | Quantity (mg/tablet) |
|---|---|
| Raloxifene HCl | 25 - 1000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

Raloxifene, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.
Suspensions each containing 0.1 - 1000 mg of medicament per 5 ml dose are made as follows:

Formulation 4: Suspensions

| Ingredient | Quantity (mg/5 ml) |
|---|---|
| Raloxifene HCl | 0.1 - 1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.
An aerosol solution is prepared containing the following ingredients:

Formulation 5: Aerosol

| Ingredient | Quantity (% by weight) |
|---|---|
| Raloxifene HCl | 0.25 |
| Ethanol | 25.75 |
| Propellant 22 (Chlorodifluoromethane) | 70.00 |

Raloxifene is mixed with ethanol and the mixture added to a portion of the propellant 22, cooled to 30° C, and transferred to a filling device. The required amount is then fed to a stainless steel container and diluted with the remaining propellant. The valve units are then fitted to the container.
Suppositories are prepared as follows:

Formulation 6: Suppositories

| Ingredient | Quantity (mg/suppository) |
|---|---|
| Raloxifene HCl | 250 |
| Saturated fatty acid glycerides | 2,000 |

Raloxifene is passed through a No. 60 mesh U.S. sieve and suspended in the saturated fatty acid glycerides previously melted using the minimal necessary heat. The mixture is then poured into a suppository mold of nominal 2 g capacity and allowed to cool.
An intravenous formulation is prepared as follows:

Formulation 7: Intravenous Solution

| Ingredient | Quantity |
|---|---|
| Raloxifene HCl | 50 mg |
| Isotonic saline | 1,000 mL |

The solution of Raloxifene is intravenously administered to a patient at a rate of about 1 mL per minute.

Formulation 8: Combination Capsule I

| Ingredient | Quantity (mg/capsule) |
|---|---|
| Raloxifene HCl | 50 |
| Premarin | 1 |
| Avicel pH 101 | 50 |
| Starch 1500 | 117.50 |
| Silicon Oil | 2 |
| Tween 80 | 0.50 |
| Cab-O-Sil | 0.25 |

Formulation 9: Combination Capsule II

| Ingredient | Quantity (mg/capsule) |
|---|---|
| Raloxifene HCl | 50 |
| Norethylnodrel | 5 |
| Avicel pH 101 | 82.50 |
| Starch 1500 | 90 |
| Silicon Oil | 2 |
| Tween 80 | 0.50 |

Formulation 10: Combination Tablet

| Ingredient | Quantity (mg/capsule) |
|---|---|
| Raloxifene HCl | 50 |
| Premarin | 1 |
| Corn Starch NF | 50 |
| Povidone, K29-32 | 6 |
| Avicel pH 101 | 41.50 |
| Avicel pH 102 | 136.50 |
| Crospovidone XL10 | 2.50 |
| Magnesium Stearate | 0.50 |
| Cab-O-Sil | 0.50 |

Formulation 11:

| Ingredient | Quantity (mg/capsule) |
|---|---|
| Raloxifene HCl | 60-150 |
| Polyvinylpyrrolidone | 15.75 |
| Polysorbate 80 | 5.25 |
| Lactose Anhydrous | 264.62 |
| Cross-linked polyvinylpyrrolidone | 31.5 |
| Stearic Acid | 5.25 |
| Magnesium Stearate | 2.63 |

The mixture of raloxifene HCl, lactose, and a portion of the cross-linked polyvinylpyrrolidone is granulated with an aqueous solution of the polyvinylpyrrolidone and polysorbate 80. The granules are dried, reduced to a suitable size, and mixed with stearic acid, magnesium stearate, and remaining cross-linked polyvinylpyrrolidone. The mixture is compressed into individual tablets.

Formulation 12:

| Ingredient | Quantity (mg/capsule) |
|---|---|
| Raloxifene HCl | 60-150 |
| Polyvinylpyrrolidone | 15.75 |
| Polysorbate 80 | 5.75 |
| Lactose Anhydrous | 132.06 |
| Dextrose | 132.06 |
| Cross-linked polyvinylpyrrolidone | 31.5 |
| Stearic Acid | 5.25 |
| Magnesium Stearate | 2.63 |

The mixture of raloxifene HCl, lactose anhydrous, dextrose, and a portion of the cross-linked polyvinylpyrrolidone is granulated with an alcoholic solution of polyvinylpyrrolidone and polysorbate 80. The granules are dried, reduced to a suitable size, and mixed with magnesium stearate, stearic acid, and remaining cross-linked polyvinylpyrrolidone. The mixture is compressed into individual tablets.

Formulation 13:

| Ingredient | Quantity (mg/capsule) |
|---|---|
| Raloxifene HCl | 60-150 |
| Hydroxypropyl Cellulose | 16.00 |
| Sodium Laurylsulfate | 10.00 |
| Dextrose | 154.00 |
| Cross-linked sodium carboxymethylcellulose | 16.00 |
| Magnesium Stearate | 4.00 |

The mixture of raloxifene HCl, dextrose, and cross-linked sodium carboxymethylcellulose is granulated with an aqueous solution of hydroxypropyl cellulose and sodium laurylsulfate. The granules are dried, reduced to a suitable size, and mixed with magnesium stearate. The mixture is compressed into individual tablets.

Formulation 14:

| Ingredient | Quantity (mg/capsule) |
|---|---|
| Raloxifene HCl | 30.00 |
| Lactose Anhydrous | 144.00 |
| Lactose, Hydrous spray Dried | 36.00 |
| Polyvinylpyrrolidone | 12.00 |
| Polysorbate 80 | 2.40 |
| Cross-linked polyvinylpyrrolidone | 14.40 |
| Magnesium Stearate | 1.20 |

The mixture of raloxifene HCl, lactose anhydrous, spray-dried hydrous lactose, and a portion of the cross-linked polyvinylpyrrolidone is granulated with an aqueous solution of polyvinylpyrrolidone and polysorbate 80. The granules are dried, reduced to a suitable size, and mixed with magnesium stearate and remaining cross-linked polyvinylpyrrolidone. The mixture is compressed into individual tablets yielding a tablet weight of 240 mg.

Formulation 15:

| Ingredient | Quantity (mg/capsule) |
|---|---|
| Raloxifene HCl | 30.00 |
| Lactose Anhydrous | 160.00 |
| Hydroxypropyl Cellulose | 11.00 |
| Poloxamer | 7.00 |
| Cross-linked sodium carboxymethylcellulose | 23.00 |
| Stearic Acid | 2.00 |
| Magnesium Stearate | 4.00 |

The mixture of raloxifene HCl, anhydrous lactose, and cross-linked sodium carboxymethylcellulose is granulated with an aqueous solution of poloxamer and hydroxypropyl cellulose. The granules are dried, reduced to a suitable size, and mixed with stearic acid and magnesium stearate. The mixture is then compressed into individual tablets yielding a tablet weight of 240 mg.

Formulation 16:

| Ingredient | Quantity (mg/capsule) |
|---|---|
| Raloxifene HCl | 30.00 |
| Lactose | 89.00 |
| Dextrose | 89.00 |
| Hydroxypropyl methylcellulose | 10.00 |
| Sodium Laurylsulfate | 5.00 |
| Cross-linked sodium polyvinylpyrrolidone | 12.00 |
| Stearic Acid | 5.00 |

The mixture of raloxifene HCl, lactose, dextrose, and cross-linked polyvinylpyrrolidone is granulated with an aqueous solution of hydroxypropyl methylcellulose and sodium laurylsulfate. The granules are dried, reduced to a suitable size, and mixed with the stearic acid. The mixture is then compressed into individual tablets yielding a tablet weight of

240 mg.

Formulation 17:

| Ingredient | Quantity (mg/capsule) |
|---|---|
| Raloxifene HCl | 60.00 |
| Lactose Anhydrous | 156.00 |
| Polyvinylpyrrolidone | 7.20 |
| Polysorbate 80 | 7.20 |
| Cross-linked sodium polyvinylpyrrolidone | 7.20 |
| Magnesium Stearate | 2.40 |

The mixture of raloxifene HCl, lactose anhydrous, and cross-linked polyvinylpyrrolidone is granulated with an aqueous solution of polyvinylpyrrolidone and polysorbate 80. The granules are dried, reduced to a suitable size, and mixed with magnesium stearate. The mixture is then compressed into individual tablets yielding a tablet weight of 240 mg.

Formulation 18:

| Ingredient | Quantity (mg/capsule) |
|---|---|
| Raloxifene HCl | 60.00 |
| Lactose Anhydrous | 120.00 |
| Lactose, hydrous spray-dried | 30.00 |
| Polyvinylpyrrolidone | 12.00 |
| Polysorbate 80 | 2.40 |
| Cross-linked sodium polyvinylpyrrolidone | 14.40 |
| Magnesium Stearate | 1.20 |

The mixture of raloxifene HCl, lactose anhydrous, spray-dried hydrous lactose, and a portion of the cross-linked polyvinylpyrrolidone is granulated with an aqueous solution of polyvinylpyrrolidone and polysorbate 80. The granules are dried, reduced to a suitable size, and mixed with magnesium stearate and remaining cross-linked polyvinylpyrrolidone. The mixture is then compressed into individual tablets yielding a tablet weight of 240 mg.

Formulation 19:

| Ingredient | Quantity (mg/capsule) |
|---|---|
| Raloxifene HCl | 60.00 |
| Mannitol | 77.00 |
| Dextrose | 73.00 |
| Hydroxypropyl methylcellulose | 7.00 |
| Polysorbate 80 | 4.00 |
| Sodium Starch Glycolate | 14.00 |
| Stearic Acid | 4.00 |
| Magnesium Stearate | 1.00 |

The mixture of raloxifene HCl, mannitol, dextrose, and sodium starch glycolate is granulated with an aqueous

solution of polysorbate 80 and hydroxypropyl methylcellulose. The granules are dried, reduced to a suitable size, and mixed with stearic acid and magnesium stearate. The mixture is then compressed into individual tablets yielding a tablet weight of 240 mg.

Formulation 20:

| Ingredient | Quantity (mg/capsule) |
|---|---|
| Raloxifene HCl | 150.00 |
| Lactose Anhydrous | 41.00 |
| Lactose, hydrous spray-dried | 10.25 |
| Polyvinylpyrrolidone | 11.50 |
| Polysorbate 80 | 2.30 |
| Cross-linked sodium polyvinylpyrrolidone | 13.80 |
| Magnesium Stearate | 1.15 |

The mixture of raloxifene HCl, anhydrous lactose, hydrous spray-dried lactose, and a portion of the cross-linked polyvinylpyrrolidone is granulated with an aqueous solution of polyvinylpyrrolidone and polysorbate 80. The granules are dried, reduced to a suitable size, and mixed with magnesium stearate and the remaining cross-linked polyvinylpyrrolidone. The mixture is then compressed into individual tablets yielding a tablet weight of 230 mg.

Formulation 21:

| Ingredient | Quantity (mg/capsule) |
|---|---|
| Raloxifene HCl | 150.00 |
| Lactose, hydrous spray-dried | 56.00 |
| Polyvinylpyrrolidone | 7.00 |
| Polysorbate 80 | 1.20 |
| Cross-linked sodium polyvinylpyrrolidone | 13.80 |
| Magnesium Stearate | 2.00 |

The mixture of raloxifene HCl, hydrous spray-dried lactose, and a portion of the cross-linked polyvinylpyrrolidone is granulated with an aqueous solution of polyvinylpyrrolidone and polysorbate 80. The granules are dried, reduced to a suitable size and mixed with magnesium stearate and remaining cross-linked polyvinylpyrrolidone. The mixture is then compressed into individual tablets yielding a tablet weight of 230 mg.

Formulation 22:

| Ingredient | Quantity (mg/capsule) |
|---|---|
| Raloxifene HCl | 150.00 |
| Lactose, anhydrous | 52.40 |
| Polyvinylpyrrolidone | 11.50 |
| Polysorbate 80 | 4.60 |
| Polyethylene Glycol 8000 | 11.50 |

The mixture of raloxifene HCl and anhydrous lactose is granulated with an aqueous solution of polysorbate 80 and polyvinylpyrrolidone. The granules are dried, reduced to a suitable size, and mixed with the polyethylene glycol 8000. The mixture is then compressed into individual tablets yielding a tablet weight of 230 mg.

Capsules may be prepared using the ingredients and procedures as described below:

Formulation 23:

| Ingredient | Quantity (mg/capsule) |
|---|---|
| Raloxifene HCl | 30.00 |
| Lactose, hydrous spray-dried | 178.30 |
| Sodium laurylsulfate | 4.60 |
| Cross-linked polyvinylpyrrolidone | 9.20 |
| Hydroxypropyl methylcellulose | 6.90 |
| Colloidal Silicon Dioxide | 1.00 |

The mixture of raloxifene HCl, hydrous spray-dried lactose, and cross-linked polyvinylpyrrolidone is granulated with an aqueous solution of sodium laurylsulfate and hydroxypropyl methylcellulose. The granules are dried, reduced to a suitable size, and mixed with colloidal silicon dioxide. This mixture is then filled into Size 3 hard-shell gelatin capsules utilizing conventional encapsulating equipment, with each capsule containing 230 mg of the final mixture.

Formulation 24:

| Ingredient | Quantity (mg/capsule) |
|---|---|
| Raloxifene HCl | 60.00 |
| Lactose, hydrous spray-dried | 148.30 |
| Sodium laurylsulfate | 4.60 |
| Cross-linked polyvinylpyrrolidone | 9.20 |
| Hydroxypropyl methylcellulose | 6.90 |
| Colloidal Silicon Dioxide | 1.00 |

The mixture of raloxifene HCl, hydrous spray-dried lactose, and cross-linked polyvinylpyrrolidone is granulated with an aqueous solution of sodium laurylsulfate and hydroxypropyl methylcellulose. The granules are dried, reduced to a suitable size, and mixed with colloidal silicon dioxide. This mixture is then filled into Size 3 hard-shell gelatin capsules utilizing conventional encapsulating equipment, with each capsule containing 230 mg of the final mixture.

Formulation 25:

| Ingredient | Quantity (mg/capsule) |
|---|---|
| Raloxifene HCl | 150.00 |
| Lactose, hydrous spray-dried | 58.30 |
| Sodium laurylsulfate | 4.60 |
| Cross-linked polyvinylpyrrolidone | 9.20 |
| Hydroxypropyl methylcellulose | 6.90 |
| Colloidal Silicon Dioxide | 1.00 |

The mixture of raloxifene HCl, hydrous spray-dried lactose, and cross-linked polyvinylpyrrolidone is granulated with an aqueous solution of sodium laurylsulfate and hydroxypropyl methylcellulose. The granules are dried, reduced

to a suitable size, and mixed with colloidal silicon dioxide. This mixture is then filled into Size 3 hard-shell gelatin capsules utilizing conventional encapsulating equipment, with each capsule containing 230 mg of the final mixture.

## Claims

**1.** A compound of formula I

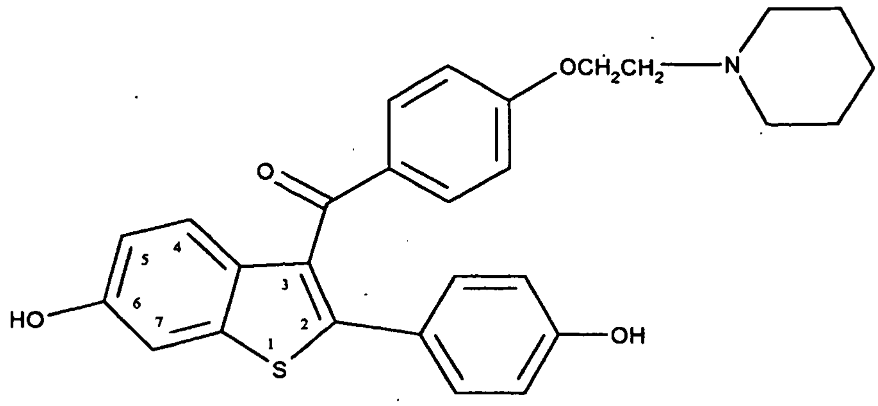

(I)

and pharmaceutically acceptable salts and solvates thereof, **characterized in that** the compound is in particulate form, said particles having a mean particle size of less than about 25 microns, at least about 90% of said particles having a size of less than about 50 microns.

**2.** The compound of Claim 1 wherein said particles have a mean particle size of between about 5 and about 20 microns.

**3.** The compound of Claim 1 or 2 wherein at least 90% of said particles have a size of less than about 35 microns.

**4.** A compound of any of Claims 1 to 3 wherein the compound of formula I is raloxifene hydrochloride.

**5.** A compound of Claim 4 which is the non-solvated crystalline hydrochloride having substantially the following X-ray diffraction pattern obtained with copper radiation:

| d-line spacing (Angstroms) | I/Io (x100) |
|---|---|
| 13.3864 | 71.31 |
| 9.3598 | 33.16 |
| 108.4625 | 2.08 |
| 7.3888 | 7.57 |
| 6.9907 | 5.80 |
| 6.6346 | 51.04 |
| 6.1717 | 29.57 |
| 15 5.9975 | 5.67 |
| 5.9135 | 9.87 |
| 5.6467 | 38.47 |
| 5.4773 | 10.54 |
| 5.2994 | 4.74 |
| 4.8680 | 4.03 |
| 4.7910 | 5.98 |
| 4.6614 | 57.50 |

(continued)

| d-line spacing (Angstroms) | I/Io (x100) |
|---|---|
| 4.5052 | 5.75 |
| 4.3701 | 9.03 |
| 4.2516 | 69.99 |
| 4.2059 | 57.64 |
| 4.1740 | 65.07 |
| 4.0819 | 12.44 |
| 3.9673 | 22.53 |
| 3.9318 | 100.00 |
| 3.8775 | 9.07 |
| 3.7096 | 33.38 |
| 3.6561 | 21.65 |
| 3.5576 | 3.36 |
| 3.5037 | 7.97 |
| 3.4522 | 18.02 |
| 3.4138 | 4.65 |
| 3.2738 | 10.23 |
| 3.1857 | 8.90 |
| 3.1333 | 6.24 |
| 3.0831 | 9.43 |
| 3.0025 | 12.13 |
| 2.9437 | 4.96 |
| 2.8642 | 7.70 |
| 2.7904 | 11.95 |
| 2.7246 | 3.05 |
| 2.6652 | 3.32 |
| 2.5882 | 7.30 |

**6.** A pharmaceutical formulation comprising a compound of any one of Claims 1 to 5 and one or more pharmaceutically acceptable carriers, diluents, or excipients.

**7.** A pharmaceutical composition comprising or a compound according to Claim 1, or a pharmaceutically acceptable salt or solvate thereof, in combination with one or more pharmaceutically acceptable carriers, diluents or excipients.

**8.** A compound according to any one of Claims 1 to 5 for use in inhibiting osteoporosis.

**9.** A compound according to any one of Claims 1 to 5 for use in lowering serum lipid levels.

**10.** A compounds according to any one of Claims 1 to 5 for use in preventing breast cancer.

**Patentansprüche**

**1.** Verbindung der Formel I

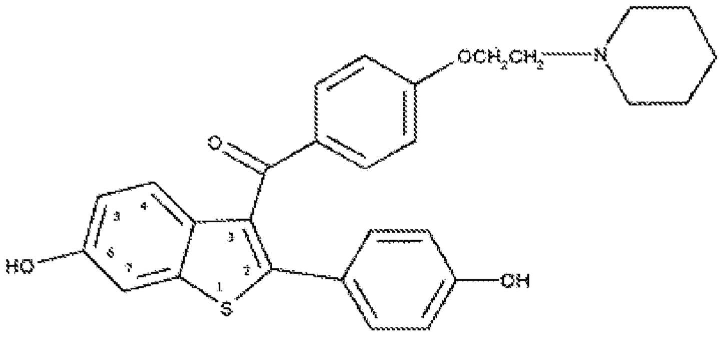

(I)

und pharmazeutisch akzeptable Salze und Solvate hiervon, **dadurch gekennzeichnet, dass** die Verbindung in teilchenförmiger Form vorliegt, die Teilchen eine mittlere Teilchengröße von weniger als etwa 25 μm aufweisen und mindestens etwa 90% der Teilchen eine Größe von weniger als etwa 50 μm aufweisen.

**2.** Verbindung nach Anspruch 1, wobei die Teilchen eine mittlere Teilchengröße von zwischen etwa 5 und etwa 20 μm aufweisen.

**3.** Verbindung nach Anspruch 1 oder 2, wobei mindestens 90% der Teilchen eine Größe von weniger als etwa 35 μm aufweisen.

**4.** Verbindung nach einem der Ansprüche 1 bis 3, wobei die Verbindung der Formel I Raloxifenhydrochlorid ist.

**5.** Verbindung nach Anspruch 4, wobei das nicht solvatisierte, kristalline Hydrochlorid im Wesentlichen das folgende, mit Kupferstrahlung erhaltene Röntgenbeugungsdiagramm aufweist:

| d-Linienabstand (Angström) | $I/I_0$ (x100) |
|---|---|
| 13,3864 | 71,31 |
| 9,3598 | 33,16 |
| 8,4625 | 2,08 |
| 7,3888 | 7,57 |
| 6,9907 | 5,80 |
| 6,6346 | 51,04 |
| 6,1717 | 29,57 |
| 5,9975 | 5,67 |
| 5,9135 | 9,87 |
| 5,6467 | 38,47 |
| 5,4773 | 10,54 |
| 5,2994 | 4,74 |
| 4,8680 | 4,03 |
| 4,7910 | 5,98 |
| 4,6614 | 57,50 |
| 4,5052 | 5,75 |
| 4,3701 | 9,03 |
| 4,2516 | 69,99 |
| 4,2059 | 57,64 |
| 4,1740 | 65,07 |
| 4,0819 | 12,44 |
| 3,9673 | 22,53 |
| 3,9318 | 100,00 |

(fortgesetzt)

| d-Linienabstand (Angström) | $I/I_0$ (x100) |
|---|---|
| 3,8775 | 9,07 |
| 3,7096 | 33,38 |
| 3,6561 | 21,65 |
| 3,5576 | 3,36 |
| 3,5037 | 7,97 |
| 3,4522 | 18,02 |
| 3,4138 | 4,65 |
| 3,2738 | 10,23 |
| 3,1857 | 8,90 |
| 3,1333 | 6,24 |
| 3,0831 | 9,43 |
| 3,0025 | 12,13 |
| 2,9437 | 4,96 |
| 2,8642 | 7,70 |
| 2,7904 | 11,95 |
| 2,7246 | 3,05 |
| 2,6652 | 3,32 |
| 2,5882 | 7,30 |

**6.** Pharmazeutische Formulierung, die eine Verbindung nach einem der Ansprüche 1 bis 5 und einen oder mehrere pharmazeutisch akzeptable Träger, Verdünnungsmittel oder Hilfsstoffe umfasst.

**7.** Pharmazeutische Zusammensetzung, die eine Verbindung nach Anspruch 1 oder ein pharmazeutisch akzeptables Salz oder Solvat hiervon in Kombination mit einem oder mehreren pharmazeutisch akzeptablen Trägern, Verdünnungsmitteln oder Hilfsträgern umfasst, oder Verbindung nach Anspruch 1 oder ein pharmazeutisch akzeptables Salz oder Solvat hiervon in Kombination mit einem oder mehreren pharmazeutisch akzeptablen Trägern, Verdünnungsmitteln oder Hilfsstoffen.

**8.** Verbindung nach einem der Ansprüche 1 bis 5 zur Verwendung bei der Hemmung von Osteoporose.

**9.** Verbindung nach einem der Ansprüche 1 bis 5 zur Verwendung bei der Verringerung der Serumlipidspiegel.

**10.** Verbindung nach einem der Ansprüche 1 bis 5 zur Verwendung bei der Verhinderung von Brustkrebs.

## Revendications

**1.** Composé répondant à la formule I

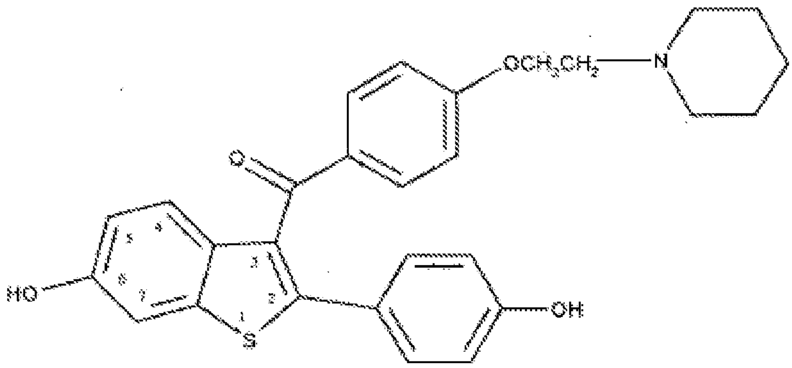

(I)

ainsi que ses sels et ses solvates pharmaceutiquement acceptables, **caractérisé en ce que** le composé est présent sous forme particulaire, lesdites particules possédant une granulométrie moyenne inférieure à environ 25 microns, au moins environ 90 % desdites particules possédant une granulométrie inférieure à environ 50 microns.

2. Composé selon la revendication 1, dans lequel lesdites particules possèdent une granulométrie moyenne entre environ 5 et environ 20 microns.

3. Composé selon la revendication 1 ou 2, dans lequel au moins 90 % desdites particules possèdent une granulométrie inférieure à environ 35 microns.

4. Composé selon l'une quelconque des revendications 1 à 3, dans lequel le composé répondant à la formule I est le chlorhydrate de raloxifène.

5. Composé selon la revendication 4, à savoir le chlorhydrate cristallin non solvaté possédant essentiellement le diagramme de diffraction des rayons X suivant, que l'on obtient avec un rayonnement du cuivre :

| Distance interréticulaire d (Angströms) | I/Io (x 100) |
|---|---|
| 13,3864 | 71,31 |
| 9,3598 | 33,16 |
| 8,4625 | 2,08 |
| 7,3888 | 7,57 |
| 6,9907 | 5,80 |
| 6,6346 | 51,04 |
| 6,1717 | 29,57 |
| 5,9975 | 5,67 |
| 5,9135 | 9,87 |
| 5,6467 | 38,47 |
| 5,4773 | 10,54 |
| 5,2994 | 4,74 |
| 4,8680 | 4,03 |
| 4,7910 | 5,98 |
| 4,6614 | 57,50 |
| 4,5052 | 5,75 |
| 4,3701 | 9,03 |
| 4,2516 | 69,99 |
| 4,2059 | 57,64 |

(suite)

| Distance interréticulaire d (Angströms) | I/Io (x 100) |
|---|---|
| 4,1740 | 65,07 |
| 4,0819 | 12,44 |
| 3,9673 | 22,53 |
| 3,9318 | 100,00 |
| 3,8775 | 9,07 |
| 3,7096 | 33,38 |
| 3,6561 | 21,65 |
| 3,5576 | 3,36 |
| 3,5037 | 7,97 |
| 3,4522 | 18,02 |
| 3,4138 | 4,65 |
| 3,2738 | 10,23 |
| 3,1857 | 8,90 |
| 3,1333 | 6,24 |
| 3,0831 | 9,43 |
| 3,0025 | 12,13 |
| 2,9437 | 4,96 |
| 2,8642 | 7,70 |
| 2,7904 | 11,95 |
| 2,7246 | 3,05 |
| 2,6652 | 3,32 |
| 2,5882 | 7,30 |

**6.** Composition pharmaceutique comprenant un composé selon l'une quelconque des revendications 1 à 5, et un ou plusieurs supports, diluants ou excipients pharmaceutiquement acceptables.

**7.** Composition pharmaceutique comprenant un composé selon la revendication 1, ou un de ses sels ou de ses solvates pharmaceutiquement acceptables, en combinaison avec un ou plusieurs supports, diluants ou excipients pharmaceutiquement acceptables.

**8.** Composé selon l'une quelconque des revendications 1 à 5, à utiliser dans l'inhibition de l'ostéoporose.

**9.** Composé selon l'une quelconque des revendications 1 à 5, à utiliser dans la diminution des taux de lipides sériques.

**10.** Composé selon l'une quelconque des revendications 1 à 5, à utiliser dans la prévention du cancer du sein.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5494920 A
- US 5494929 A
- US 5532254 A
- US 4133814 A
- US 4418068 A
- US 4380635 A
- GB 2293382 A
- DE 19534744
- US 4605517 A